# EUROPÄISCHE PATENTSCHRIFT

(11) **EP 2 419 162 B1**
(45) Veröffentlichungstag und Bekanntmachung des Hinweises auf die Patenterteilung: **16.01.2013**
(21) Anmeldenummer: 09777554.8
(22) Anmeldetag: 30.07.2009
(51) Int. Cl.: A45D 34/04

(54) **FLÜSSIGKEITSPUMPENGERÄT**
LIQUID PUMP APPLIANCE
APPAREIL DE POMPAGE DE LIQUIDE

(30) Priorität: 13.04.2009 WO PCT/EP2009/002810; 13.04.2009 WO PCT/EP2009/002807
(43) Veröffentlichungstag der Anmeldung: 22.02.2012
(73) Patentinhaber: Dadgar, Karan, 22765 Hamburg (DE)
(72) Erfinder: Dadgar, Karan, 22765 Hamburg (DE)
(74) Vertreter: Heun, Thomas
(86) Internationale Anmeldenummer: PCT/EP2009/005532
(87) Internationale Veröffentlichungsnummer: WO 2010/118764

(56) Entgegenhaltungen:
- EP-A2- 0 420 047
- DE-U1- 9 107 574
- DE-U1- 9 412 461

## Beschreibung

Die Erfindung betrifft ein Flüssigkeitspumpengerät in Form einer spritzenartigen Vorrichtung, einer Injektionsspritze oder eines Spenders, zum dosierten Abgeben bzw. Injizieren von Fluiden wie insbesondere Flüssigkeiten, auch in Form eines Sprühnebels, oder flüssigen oder breiigen Massen oder Pasten, mit einem Vorratsbehälter für das Fluid sowie einer Abgabeeinrichtung zum dosierten Abgeben des Fluids. Die Erfindung eignet sich insbesondere zum dosierten Abgeben von Farbstoffen, Klebstoffen und Dichtungsmassen sowie Lebensmittelmassen wie Gewürzsaucen und Brotaufstrichen, bzw. zum dosierten Injizieren von zum Beispiel Impfstoffen, Medikamenten oder anderen zum Beispiel zahnmedizinischen Substanzen.

Aus der DE-G 94 12 461 ist ein Auftragsgerät bekannt, bei dem eine flüssige Masse durch den Druck eines federbelasteten Kolbens abgegeben wird, wenn durch Eindrücken eines Betätigungsknopfes eine Ringnut eines verschiebbaren Ventilkörpers in Fluchtung mit Durchtrittskanälen für die flüssige Masse gebracht wird.

In der DE-G 91 07 574 wird eine Vorrichtung beschrieben, die einen Hebel aufweist, mit dem durch Ziehen ein Verbindungsrohr sowie ein Kolben in Richtung einer Auslassöffnung eines Auftragsgerätes verschoben wird, wodurch sich ein Ventil öffnet und ein entsprechend dem Vorschub des Kolbens verdrängtes Volumen an Flüssigkeit aus einer Kammer abgegeben wird. Beim Loslassen des Hebels drückt eine Rückstellfeder das Verbindungsrohr zusammen mit dem Kolben wieder in die Ausgangsposition zurück, so dass das Ventil schließt und ein weiteres Ventil öffnet, durch das wieder Flüssigkeit in die Kammer gesaugt wird.

Nachteilig bei diesen bekannten Vorrichtungen ist, dass sie aus einer Vielzahl von Einzelteilen zusammengesetzt und somit konstruktiv relativ aufwändig und teuer in der Herstellung sind.

Eine Aufgabe, die der Erfindung zugrunde liegt, besteht deshalb darin, ein Flüssigkeitspumpengerät der eingangs genannten Art zu schaffen, mit dem bei konstruktiv relativ einfachem und somit kostengünstigem Aufbau eine genaue Dosierung des abgegebenen Fluids in einfacher und zuverlässiger Weise möglich ist.

Gelöst wird diese Aufgabe gemäß Anspruchs 1 mit einem Flüssigkeitspumpengerät der eingangs genanten Art, mit einem Vorratsbehälter für das Fluid sowie einer Abgabeeinrichtung für das Fluid, wobei die Abgabeeinrichtung ein Pumpenröhrchen aufweist, in dem mittels eines hermetisch in diesem geführten Kolbens ein Volumen für das Fluid abgegrenzt wird, wobei der Kolben zur Veränderung des Volumens entlang des Pumpenröhrchens verschiebbar ist, und wobei das Fluid bei einer Vergrößerung des Volumens durch ein erstes Rückschlagventil aus dem Vorratsbehälter angesaugt, und bei einer Verkleinerung des Volumens durch ein zweites Rückschlagventil aus dem Volumen und durch ein Abflussröhrchen aus dem Flüssigkeitspumpengerät abgegeben wird.

Ein besonderer Vorteil dieser Lösung besteht darin, dass das Flüssigkeitspumpengerät einfach und zuverlässig handhabbar ist, wobei in jeder Ausrichtung des Gerätes, also auch in einer Abgaberichtung nach oben, eine genau dosierte Abgabe des Fluids möglich ist, so dass der Arbeitsablauf auch unter schwierigen Bedingungen erleichtert und beschleunigt wird. Dies gilt sogar für eine Anwendung des Flüssigkeitspumpengerätes unter schwerelosen Bedingungen.

Die Unteransprüche haben vorteilhafte Weiterbildungen der Erfindung zum Inhalt.

Mit den Ausführungsformen gemäß den Ansprüchen 2, 3, 4 und 5 wird die Handhabung und Betätigung des erfindungsgemäßen Flüssigkeitspumpengerätes weiter erleichtert.

Die Ausführungsformen gemäß den Ansprüchen 6 und 7 haben insbesondere den Vorteil, dass die abzugebende Menge des Fluids sehr genau und zuverlässig dosiert werden kann.

Mit den Ausführungsformen gemäß den Ansprüchen 8, 9 und 10 ist eine zumindest nahezu vollständige Entleerung des Vorratsbehälters möglich, so dass keine unverbrauchbaren Reste des Fluids in dem Vorratsbehälter verbleiben und entsorgt werden müssen.

Weitere Einzelheiten, Merkmale und Vorteile der Erfindung ergeben sich aus der folgenden Beschreibung von beispielhaften und bevorzugten Ausführungsformen anhand der Figuren, in denen gleiche oder einander entsprechende Teile oder Komponenten jeweils mit gleichen Bezugsziffern bezeichnet sind. Es zeigt:
- Fig. 1: eine schematische Gesamtansicht einer ersten Ausführungsform eines erfindungsgemäßen Flüssigkeitspumpengerätes;
- Fig. 2: eine schematische Detaildarstellung des Bereiches der Abgabeeinrichtung des Flüssigkeitspumpengerätes gemäß Figur 1;
- Fig. 3: eine schematische Ansicht eines Pumpenröhrchens gemäß der ersten Ausführungsform in einer ersten Abwandlung;
- Fig. 4: eine schematische Ansicht eines Pumpenröhrchens gemäß der ersten Ausführungsform in einer zweiten Abwandlung;
- Fig. 5: eine schematische Ansicht eines Pumpenröhrchens gemäß der ersten Ausführungsform in einer dritten Abwandlung;
- Fig. 6: eine schematische Detaildarstellung des Bereiches der Abgabeeinrichtung eines Flüssigkeitspumpengerätes gemäß einer zweiten Ausführungsform; und
- Fig. 7: ein beispielhaftes Auftragsorgan für ein erfindungsgemäßes Flüssigkeitspumpengerät.

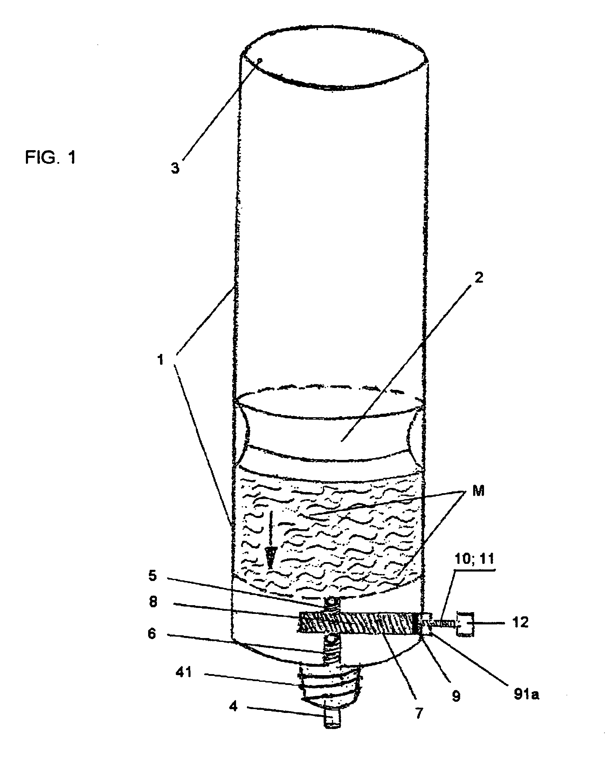

Figur 1 zeigt schematisch eine Gesamtansicht eines erfindungsgemäßen Flüssigkeitspumpengerätes gemäß einer ersten Ausführungsform. Das Gerät umfasst einen Vorratsbehälter 1, in dem sich das durch ein Abflussröhrchen 4 abzugebende bzw. zu injizierende Fluid M befindet. Der Vorratsbehälter 1 ist vorzugsweise zylindrisch, kann aber auch andere Formen aufweisen. Innerhalb des Vorratsbehälters 1 befindet sich vorzugsweise ein Vorratsbehälter-Kolben 2, der auf dem Fluid M ruht und hermetisch mit der Innenwand des Vorratsbehälters 1 abschließt. Im oberen Bereich des Vorratsbehälters 1, d.h. an dessen dem Abflussröhrchen 4 gegenüberliegenden Ende, befindet sich eine kleine Öffnung 3, durch die Luft in den Raum des Vorratsbehälters 1 oberhalb des Vorratsbehälter-Kolbens 2 eintreten kann, um während der Entleerung des Vorratsbehälters 1 einen Druckausgleich zu schaffen.

Das Flüssigkeitspumpengerät umfasst ferner eine Abgabeeinrichtung, die im Wesentlichen ein Pumpenröhrchen 7 sowie ein erstes Rückschlagventil 5 zur Zuführung von Fluid aus dem Vorratsbehälter 1 in das Pumpenröhrchen 7 und ein zweites Rückschlagventil 6 zur Abgabe von Fluid aus dem Pumpenröhrchen 7 in das Abflussröhrchen 4 aufweist, wobei sich in dem Pumpenröhrchen 7 ein hermetisch mit der Innenwand des Pumpenröhrchens 7 abschließender Kolben 9 befindet, mit dem innerhalb des Pumpenröhrchens 7 ein Volumen für das Fluid M abgegrenzt wird. Der Kolben 9 ist zur Veränderung dieses Volumens in dem Pumpenröhrchen 7 verschiebbar, wobei das Fluid M bei einer Vergrößerung des Volumens durch das erste Rückschlagventil 5 aus dem Vorratsbehälter 1 in das Volumen angesaugt und bei einer Verkleinerung des Volumens durch das zweite Rückschlagventil 6 aus dem Volumen und durch das Abflussröhrchen 4 aus dem Flüssigkeitspumpengerät abgegeben wird.

Der Kolben 9 wird vorzugsweise durch die Kraft einer Druck- oder Zugfeder 8 beaufschlagt, mit der dieser in einer der beiden Richtungen verschoben wird, während die Verschiebung des Kolbens 9 in der jeweils anderen Richtung manuell mittels eines mit dem Kolben 9 verbundenen Betätigungsknopfes 12 erfolgt, und zwar entweder durch Drücken, vorzugsweise bis der Betätigungsknopf 12 an einem ersten Anschlag 91a an dem Pumpenröhrchen 7 anliegt, oder durch Ziehen an dem Betätigungsknopf 12, vorzugsweise bis der Kolben 9 an diesem ersten Anschlag 91a oder einem anderen Anschlag innerhalb des Pumpenröhrchens 7 anliegt.

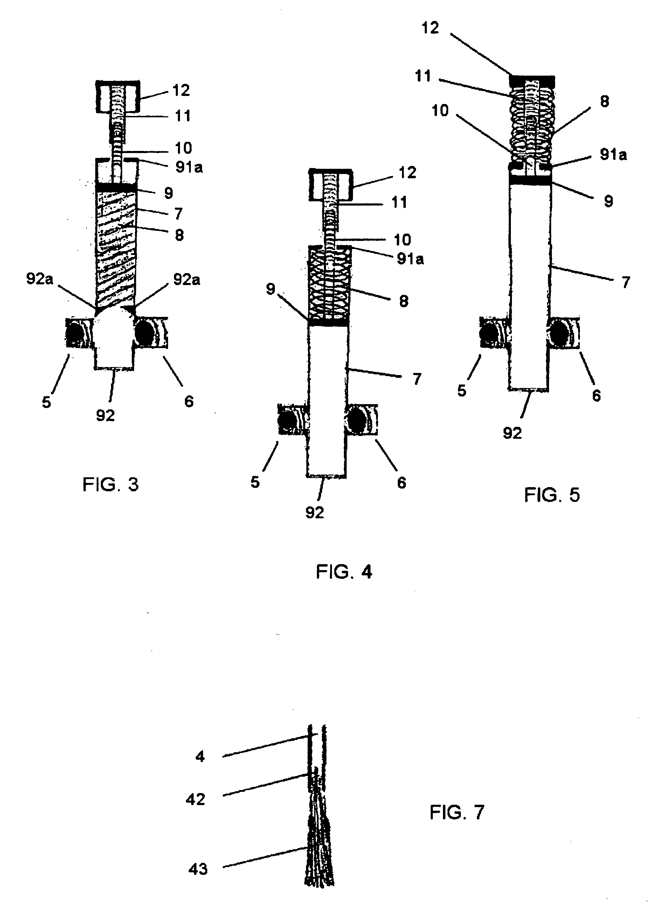

Wie im Einzelnen weiter unten noch beschrieben werden wird, kann die Druck- oder Zugfeder 8 dabei an drei verschiedenen Stellen angebracht sein, nämlich entweder, wie in Figur 1 dargestellt, zwischen dem Kolben 9 und dem dem ersten Anschlag 91a gegenüberliegenden Ende 92 des Pumpenröhrchens 7, oder zwischen dem Kolben 9 und dem ersten Anschlag 91a (siehe Figur 4), oder zwischen dem Betätigungsknopf 12 und dem ersten Anschlag 91a (siehe Figur 5).

Auf das Abflussröhrchen 4 kann vorzugsweise ein für die betreffende Anwendung bzw. das abzugebende Fluid geeignetes Auftragsorgan z.B. in Form einer Injektionsnadel, eines Pinsels oder einer Düse aufgesetzt oder mittels eines Gewindes 41 aufgeschraubt werden.

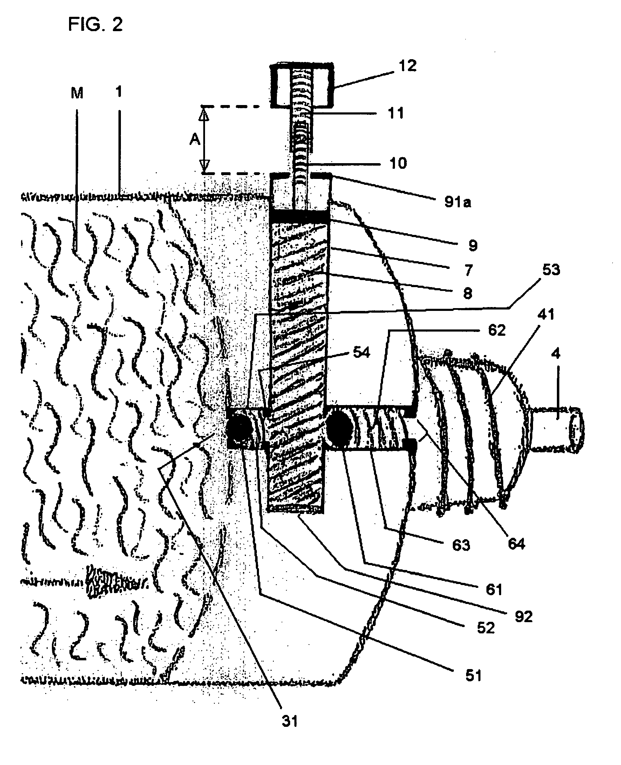

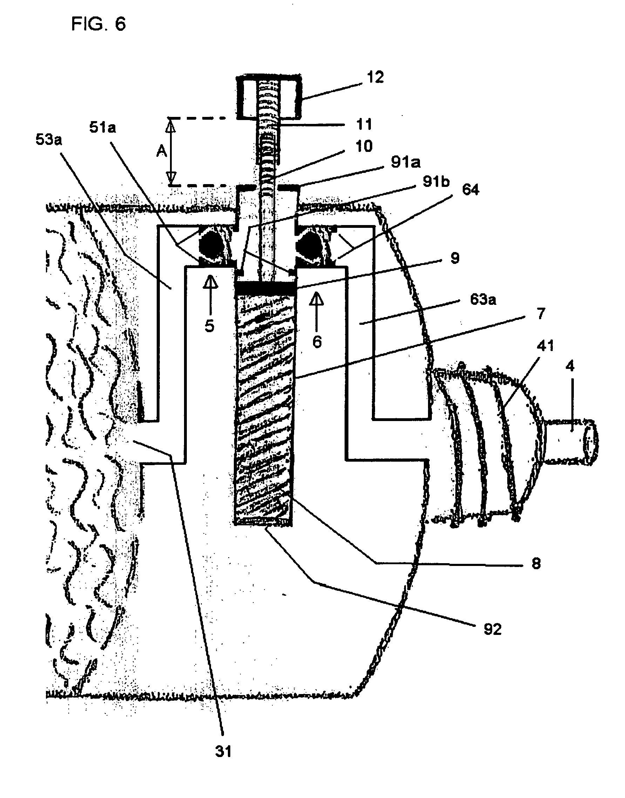

Die genannten Komponenten der Abgabeeinrichtung befinden sich in einem Bereich des Flüssigkeitspumpengerätes unterhalb des Vorratsbehälters 1, so dass sich ein relativ einfacher Aufbau und eine einfache Handhabung (ähnlich wie bei einem Schreibgerät) ergibt. Der Boden des Vorratsbehälters 1 weist eine Öffnung 31 (siehe Figuren 2 und 6) auf, durch die das Fluid M in die Abgabeeinrichtung eingesaugt werden kann.

Die Komponenten der Abgabeeinrichtung gemäß der ersten Ausführungsform sollen im Folgenden anhand der Detaildarstellung der Figur 2 beschrieben werden. In dieser Figur ist neben der Abgabeeinrichtung nur der Bodenbereich des Vorratsbehälters 1 mit dem abzugebenden Fluid M dargestellt. Gleiche Teile bzw. Komponenten wie in Figur 1 sind in Figur 2 mit den gleichen Bezugsziffern bezeichnet.

Das erste und das zweite Rückschlagventil 5, 6 ist jeweils in Form eines ersten bzw. zweiten Kugel-Federsystems ausgebildet. Im Einzelnen umfasst das erste Kugel-Federsystem 5 eine Kugel 51 sowie eine Feder 52, die in einem Röhrchen 53 geführt sind, dessen erste Öffnung in der Öffnung 31 in dem Boden des Vorratsbehälters 1 und dessen zweite Öffnung in einer ersten Öffnung in der Wand des Pumpenröhrchens 7 liegt. Die Feder 52 ist eine Druckfeder und drückt mit einem Ende die Kugel 51 gegen die ggf. einen Dichtungsring für die Kugel 51 aufweisende Öffnung 31 in dem Boden des Vorratsbehälters 1, so dass diese dadurch für das Fluid verschlossen wird. Mit ihrem anderen Ende ruht die Feder 52 an einem Absatz oder einem Widerlager 54 in dem Röhrchen 53.

Das zweite Kugel-Federsystem 6 ist in gleicher Weise aufgebaut und umfasst eine Kugel 61 sowie eine Druckfeder 62, die in einem Röhrchen 63 geführt sind. Eine erste Öffnung des Röhrchens 63 liegt in einer zweiten Öffnung in der Wand des Pumpenröhrchens 7, während über eine zweite Öffnung des Röhrchens 63 eine Fluid-Verbindung zu dem Abflussröhrchen 4 hergestellt wird. Die Druckfeder 62 wirkt zwischen der Kugel 61 und einem Absatz oder einem Widerlager 64 innerhalb des Röhrchens 63, so dass die Kugel 61 gegen die zweite, ggf. einen Dichtungsring für die Kugel 61 aufweisende Öffnung in der Wand des Pumpenröhrchens 7 gedrückt wird, so dass diese dadurch für das Fluid verschlossen wird.

Eine weitere Komponente der Abgabeeinrichtung stellt das Pumpenröhrchen 7 mit dem ersten Anschlag 91a an einem ersten Ende sowie dem diesem gegenüberliegenden zweiten Ende 92 dar, in dem die Feder 8 sowie der Kolben 9 geführt ist, wobei die Feder 8 vorzugsweise eine Druckfeder ist und zwischen dem zweiten Ende 92 des Pumpenröhrchens 7 und dem Kolben 9 wirkt und somit einen Druck auf den Kolben 9 in Richtung auf den ersten Anschlag 91a ausübt.

Alternativ dazu kann die Feder 8 gemäß der Darstellung des Pumpenröhrchens 7 in Figur 3 auch zwischen einem Absatz oder einem Widerlager 92a und dem Kolben 9 wirken, wobei der Absatz oder das Widerlager 92a an der Innenwand des Pumpenröhrchens 7 zwischen den Öffnungen für das erste und das zweite Rückschlagventil 5, 6 einerseits und dem Kolben 9 liegt, so dass sich die Feder 8 nicht über die Öffnungen für die Rückschlagventile 5, 6 erstreckt und der Kolben 9 auch nicht bis über diese Öffnungen verschoben werden kann.

Eine weitere Alternative besteht darin, zwischen dem Kolben 9 und dem ersten Anschlag 91a gemäß der Darstellung des Pumpenröhrchens 7 in Figur 4 eine Zugfeder 8 anzubringen, so dass diese den Kolben 9 in der gleichen Richtung wie die in Figur 2 gezeigte Druckfeder verschieben würde, nämlich in Richtung auf den ersten Anschlag 91a. Diese Alternative hätte den Vorteil, dass das Volumen für das Fluid frei bleiben würde und die Feder 8 nicht dem Fluid ausgesetzt wäre.

Der Kolben 9 kann mittels eines gegenüber dem ersten Anschlag 91a und außerhalb des Pumpenröhrchens 7 angeordneten und mit dem Kolben 9 verbundenen Betätigungsknopfes 12 entgegen der Kraft der Druckfeder 8 (oder der zuvor genannten Zugfeder) in Richtung auf das zweite Ende 92 des Pumpenröhrchens 7 gedrückt werden. Zur Einstellung der Weglänge, mit der der Kolben 9 eingedrückt werden kann, ist dieser vorzugsweise über einen Gewindemechanismus 10, 11 mit dem Betätigungsknopf 12 verbunden. Der Gewindemechanismus umfasst eine Gewindestange 10, die in eine Gewindehülse 11 eingedreht bzw. aus dieser herausgedreht werden kann, wobei im dargestellten Fall die Gewindestange 10 an dem Kolben 9 und die Gewindehülse 11 an dem Betätigungsknopf 12 befestigt ist. Vorzugsweise sind die Gewindestange 10 und die Gewindehülse 11 so miteinander gesichert, dass die Gewindehülse 11 nicht vollständig von der Gewindestange 10 abgedreht werden kann.

Durch Drehen des Betätigungsknopfes 12 und damit der Gewindehülse 11 gegenüber der Gewindestange 10 kann die in Figur 2 mit "A" bezeichnete maximale Weglänge des Betätigungsknopfes 12 bis zu dem ersten Anschlag 91a an dem Pumpenröhrchen 7, und damit auch die maximale Wegstrecke, mit der der Kolben 9 durch Drücken auf den Betätigungsknopf 12 in dem Pumpenröhrchen 7 in Richtung auf dessen zweites Ende 92 verschoben werden kann, vergrößert bzw. verkleinert werden.

Durch vollständiges Eindrehen des Betätigungsknopfes 12 bis an den ersten Anschlag 91a kann die Funktion der Abgabeeinrichtung außer Betrieb gesetzt werden, da in diesem Fall der Betätigungsknopf 12 nicht mehr (weiter) eingedrückt werden kann. Damit wird in wirksamer Weise verhindert, dass z.B. beim Transport des Flüssigkeitspumpengerätes versehentlich durch Druck auf den Betätigungsknopf 12 Fluid abgegeben wird.

Der erste Anschlag 91a ist mit einer Bohrung für die Gewindestange 10 versehen und dient auf diese Weise auch zur Führung der Gewindestange 10.

Insgesamt kann also durch Drehen des Betätigungsknopfes 12 die Menge des abzugebenden Fluids sehr einfach und genau eingestellt werden. Die Abgabe selbst erfolgt dann durch Eindrücken des Betätigungsknopfes 12 bis zum ersten Anschlag 91a, wodurch mittels des hermetisch an der Innenwand des Pumpenröhrchens 7 abschließend geführten Kolbens 9 ein entsprechender Druck auf das in dem Pumpenröhrchen 7 vorhandene Fluid ausgeübt wird und dadurch das zweite Kugel-Federsystem 6 öffnet, so dass das Fluid durch das Abflussröhrchen 4 austreten kann. Durch den Druck wird gleichzeitig das erste Kugel-Federsystem 5 geschlossen gehalten, so dass das Fluid nicht in den Vorratsbehälter 1 zurückströmen kann.

Wenn man dann den Betätigungsknopf 12 loslässt, wird der Kolben 9 durch die Kraft der Druckfeder 8 (oder der zuvor genannten Zugfeder) wieder in seine Ausgangslage zurückgeschoben, bis er zumindest im Wesentlichen an dem ersten Anschlag 91a anliegt. Durch den dadurch in dem Pumpenröhrchen 7 entstehenden Unterdruck schließt das zweite Kugel-Federsystem 6, und das erste Kugel-Federsystem 5 öffnet sich, so dass Fluid M aus dem Vorratsbehälter 1 in das Pumpenröhrchen 7 eingesaugt wird. Anschließend kann dann erneut Fluid in der oben beschriebenen Weise durch das Abflussröhrchen 4 abgegeben werden.

Anstelle der oben beschriebenen Druckfeder 8 könnte auch eine Zugfeder zwischen dem Kolben 9 und dem zweiten Ende 92 des Pumpenröhrchens 7 oder eine Druckfeder zwischen dem Kolben 9 und dem ersten Anschlag 91a (siehe Figur 4) verwendet werden, wobei in diesen Fällen das Einsaugen von Fluid aus dem Vorratsbehälter in das Pumpenröhrchen 7 durch manuelles Herausziehen des Betätigungsknopfes 12 und die Abgabe des Fluids M durch die Kraft einer dieser Federn erfolgen würde.

Als weitere Alternative ist es schließlich auch möglich, eine Druck- oder Zugfeder 8 zwischen dem ersten Anschlag 91a und dem Betätigungsknopf 12 anzuordnen, so wie es in Figur 5 schematisch angedeutet ist, so dass sich die Feder 8 nicht innerhalb des Pumpenröhrchens 7 befindet und im Bedarfsfall durch Abdrehen des Betätigungsknopfes 12 leicht ausgetauscht werden könnte.

Bei der in den Figuren 1 bis 5 gezeigten ersten Ausführungsform der Abgabeeinrichtung befindet sich das hinsichtlich seiner Größe veränderbare Volumen, in das das Fluid M durch Vergrößerung des Volumens eingesaugt und anschließend durch Verkleinerung des Volumens wieder abgegeben wird, zwischen dem Kolben 9 und dem dem ersten Anschlag 91a gegenüberliegenden zweiten Ende 92 des Pumpenröhrchens 7. Somit befinden sich auch die Öffnungen in dem Pumpenröhrchen 7 für das erste und das zweite Rückschlagventil 5, 6 zwischen dem Kolben 9 und dem zweiten Ende 92 des Pumpenröhrchens 7.

Figur 6 zeigt die Komponenten der Abgabeeinrichtung einer zweiten Ausführungsform des erfindungsgemäßen Flüssigkeitspumpengerätes. Gleiche Komponenten wie bei der ersten Ausführungsform sind in Figur 6 mit gleichen Bezugsziffern bezeichnet. Der wesentliche Unterschied im Vergleich zu der in den Figuren 1 bis 5 gezeigten ersten Ausführungsform besteht darin, dass sich bei der zweiten Ausführungsform die Öffnungen in dem Pumpenröhrchen 7 für das erste und das zweite Rückschlagventil 5, 6 zwischen dem ersten Anschlag 91a und dem Kolben 9 befinden, wobei vorzugsweise zwischen diesen Öffnungen und dem Kolben 9 innerhalb des Pumpenröhrchens 7 ein zweiter Anschlag 91b für den Kolben 9 angeordnet ist, so dass der Kolben 9 nicht vor diese Öffnungen gelangen kann.

Bei dieser zweiten Ausführungsform befindet sich somit das hinsichtlich seiner Größe veränderbare Volumen für das Fluid zwischen dem Kolben 9 und dem ersten Anschlag 91a. Folglich wird dieses Volumen durch ein Eindrücken oder Einziehen des Kolbens 9 in Richtung auf das zweite Ende 92 des Pumpenröhrchens 7 vergrößert und dadurch Fluid durch das erste Rückschlagventil 5 aus dem Vorratsbehälter 1 in das Volumen angesaugt, während durch ein Herausdrücken oder Herausziehen des Kolbens 9 in der entgegengesetzten Richtung dieses Volumen verkleinert und dadurch das Fluid aus dem Volumen durch das zweite Rückschlagventil 6 und das Abflussröhrchen 4 abgegeben wird. Gegebenenfalls könnte man die Abgabe des Fluids durch einen an dem Betätigungsknopf 12 angreifenden Hebelmechanismus (nicht dargestellt) erleichtern.

Auch bei dieser zweiten Ausführungsform wird der Kolben 9 vorzugsweise durch die Kraft einer Druck- oder Zugfeder 8 beaufschlagt, mit der dieser in einer der beiden Richtungen verschoben wird, während die Verschiebung des Kolbens 9 in der jeweils entgegengesetzten Richtung manuell durch Drücken auf oder Ziehen an dem Betätigungsknopf 12 erfolgt.

Die Druck- oder Zugfeder 8 kann dabei wiederum, wie oben mit Bezug auf die Figuren 2, 4 und 5 beschrieben wurde, an drei verschiedenen Stellen angebracht sein, d.h. entweder zwischen dem Kolben 9 und dem zweiten Ende 92 des Pumpenröhrchens 7 (wie in Figur 6 dargestellt), oder zwischen dem Kolben 9 und dem ersten Anschlag 91a (siehe Figur 4), oder zwischen dem ersten Anschlag 91a und dem Betätigungsknopf 12 (siehe Figur 5). Insoweit wird auf die obigen Erläuterungen im Zusammenhang mit der ersten Ausführungsform Bezug genommen.

Die Druck- oder Zugfeder 8 befindet sich gemäß der Darstellung in Figur 6 vorzugsweise zwischen dem Kolben 9 und dem zweiten Ende 92 des Pumpenröhrchens 7, so dass sie nicht dem Fluid in dem Volumen zwischen dem Kolben 9 und dem ersten Anschlag 91a ausgesetzt ist.

Der Vollständigkeit halber sei noch darauf hingewiesen, dass bei beiden Ausführungsformen auf eine Druck- oder Zugfeder 8, die auf den Kolben 9 bzw. den Betätigungsknopf 12 einwirkt, auch gänzlich verzichtet werden könnte, wobei in diesem Fall die Bewegung des Kolbens 9 in beiden Richtungen manuell erfolgt.

Das erste und das zweite Rückschlagventil 5, 6 ist wiederum in Form eines ersten bzw. zweiten Kugel-Federsystems ausgebildet, deren Teile, Funktionen und Wirkungen hinsichtlich der Durchlass- und Sperrrichtung für das Fluid die gleichen sind, wie sie oben in Bezug auf Figur 2 und die erste Ausführungsform beschrieben wurden. Auch ergänzend zu der Darstellung in Figur 2 ist in Figur 6 ein Ventilsitz 51a für die Kugel 51 des ersten Rückschlagventils 5 bezeichnet, gegen den die Kugel 51 mittels der Feder 52 (siehe Figur 2) zum Verschließen des Rückschlagventils 5 gedrückt wird. Ein solcher Sitz ist natürlich auch bei dem zweiten Rückschlagventil 6 vorhanden. Da die Rückschlagventile jedoch in an sich bekannter Weise ausgebildet sind, erübrigt sich eine detailliertere Darstellung von deren Einzelteilen.

Figur 6 zeigt schließlich noch ein erstes Verbindungsrohr 53a zur Fluid-Verbindung der Öffnung 31 des Vorratsbehälters 1 mit dem ersten Rückschlagventil 5 sowie ein zweites Verbindungsrohr 63a zur Fluid-Verbindung des zweiten Rückschlagventils 6 mit dem Abflussröhrchen 4.

Der sich im Zuge der Entnahme von Fluid M aus dem Vorratsbehälter 1 mit absenkende Vorratsbehälter-Kolben 2 bewirkt, dass der Vorratsbehälter 1 zumindest nahezu vollständig entleert werden kann. Dies trifft insbesondere dann zu, wenn die der Bodenfläche des Vorratsbehälters 1 gegenüberliegende (Boden-) Fläche des Vorratsbehälter-Kolbens 2 hinsichtlich ihrer Form und ihres Verlaufes an die Bodenfläche des Vorratsbehälters 1 angepasst ist, so dass beide zumindest ohne wesentliche Zwischenräume aufeinander zu liegen kommen, wenn sich der Vorratsbehälter 1 leert und dadurch auch kleine Reste des Fluids aus diesem herausgedrückt werden können.

Aufgrund der Tatsache, dass sich das Pumpenröhrchen 7 mit seiner Länge im Wesentlichen senkrecht zur Längsrichtung des Flüssigkeitspumpengerätes erstreckt und im Bereich von dessen abgabeseitigem Ende, d. h. unterhalb des Vorratsbehälters 1, angeordnet ist, kann das Flüssigkeitspumpengerät leicht und bequem gehandhabt werden, indem es z.B. wie ein Bleistift gehalten und der Betätigungsknopf 12 mit dem Daumen betätigt wird.

Figur 7 zeigt beispielhaft ein Auftragsorgan in Form eines Pinsels 43, der an der Öffnung des Abflussröhrchens 4 befestigt ist und der z.B. mittels einer Kanüle 42, die innerhalb des Abflussröhrchens 4 angeordnet ist und mittig innerhalb des Pinsels 43 endet, gleichmäßig von innen mit Fluid getränkt wird, so dass dieses gleichmäßig auf einen Gegenstand aufgetragen werden kann.

## Patentansprüche

1. Flüssigkeitspumpengerät zum dosierten Abgeben bzw. Injizieren von Fluiden, mit einem Vorratsbehälter (1) für das Fluid (M) sowie einer Abgabeeinrichtung für das Fluid (M), wobei die Abgabeeinrichtung ein Pumpenröhrchen (7) aufweist,
**dadurch gekennzeichnet, dass** in dem Pumpenröhrchen (7) mittels eines hermetisch abschließend in diesem geführten Kolbens (9) ein Volumen für das Fluid (M) abgegrenzt wird, wobei der Kolben (9) zur Veränderung dieses Volumens entlang des Pumpenröhrchens (7) verschiebbar ist, und wobei das Fluid (M) bei einer Vergrößerung des Volumens durch ein erstes Rückschlagventil (5) aus dem Vorratsbehälter (1) in dieses Volumen eingesaugt, und bei einer Verkleinerung des Volumens durch ein zweites Rückschlagventil (6) aus diesem Volumen und durch ein Abflussröhrchen (4) aus dem Flüssigkeitspumpengerät abgegeben wird.

2. Flüssigkeitspumpengerät nach Anspruch 1,
wobei das Pumpenröhrchen (7) sowie das erste und das zweite Rückschlagventil (5, 6) zwischen dem Boden des Vorratsbehälters (1) und dem Abflussröhrchen (4) angeordnet sind.

3. Flüssigkeitspumpengerät nach Anspruch 2,
bei dem sich das Pumpenröhrchen (7) mit seiner Länge im Wesentlichen senkrecht zu der Längserstreckung des Flüssigkeitspumpengerätes erstreckt.

4. Flüssigkeitspumpengerät nach Anspruch 1,
mit einem mit dem Kolben (9) verbundenen Betätigungsknopf (12) zur manuellen Verschiebung des Kolbens (9) in dem Pumpenröhrchen (7) in einer ersten Richtung.

5. Flüssigkeitspumpengerät nach Anspruch 4,
mit einer auf den Kolben (9) oder den Betätigungsknopf (12) einwirkenden Druck- oder Zugfeder (8), mit der der Kolben (9) in einer der ersten Richtung entgegengesetzten zweiten Richtung in dem Pumpenröhrchen (7) verschiebbar ist.

6. Flüssigkeitspumpengerät nach Anspruch 4,
bei dem der Betätigungsknopf (12) über einen Gewindemechanismus mit dem Kolben (9) verbunden ist, wobei der Gewindemechanismus eine Gewindestange (10) sowie eine Gewindehülse (11) aufweist, die zur Veränderung der Länge des Gewindemechanismus zwischen dem Betätigungsknopf (12) und einem ersten Anschlag (91a) für den Betätigungsknopf (12), und damit zur Einstellung eines maximalen Verschiebeweges des Kolbens (9), gegeneinander verdrehbar sind.

7. Flüssigkeitspumpengerät nach Anspruch 1,
bei dem sich das Volumen für das Fluid innerhalb des Pumpenröhrchens (7) zwischen dem Kolben (9) und einem ersten oder einem diesem gegenüberliegenden zweiten Ende (91a; 92) des Pumpenröhrchens (7) befindet.

8. Flüssigkeitspumpengerät nach Anspruch 1,
bei dem das erste Rückschlagventil (5) mit einer Öffnung (31) in der Bodenfläche des Vorratsbehälters (1) Fluid-verbunden ist.

9. Flüssigkeitspumpengerät nach Anspruch 1,
bei dem der Vorratsbehälter (1) einen Vorratsbehälter-Kolben (2) aufweist, der hermetisch in diesem in der Weise geführt ist, dass er auf dem Fluid ruht und sich mit sinkendem Spiegel des Fluids mit diesem abgesenkt.

10. Flüssigkeitspumpengerät nach Anspruch 9,
bei dem die der Bodenfläche des Vorratsbehälters (1) gegenüberliegende Fläche des Vorratsbehälter-Kolbens (2) hinsichtlich ihrer Form und ihres Verlaufes an die Bodenfläche des Vorratsbehälters (1) angepasst ist.

11. Flüssigkeitspumpengerät nach Anspruch 1,
bei dem der Vorratsbehälter (1) eine Öffnung (3) aufweist, die zur Luftzufuhr dient, wenn dem Vorratsbehälter (1) durch die Abgabeeinrichtung Fluid entnommen wird.

## Claims

1. Liquid pump appliance for dosed dispense or injection of fluids, comprising a supply vessel (1) for the fluid (M) and a dispense device for the fluid (M), wherein the dispense device comprises a pump tube (7),
**characterized in that** a volume for the fluid (M) is defined within the pump tube (7) by means of a piston (9) which is guided hermetically sealed within the pump tube, wherein the piston (9) is slidable along the pump tube (7) for varying this volume, and wherein the fluid (M) is drawn from the supply vessel (1) through a first non-return valve (5) into this volume when the volume is increased, and is dispensed from this volume through a second non-return valve (6) and through a discharge tube (4) out of the liquid pump appliance when the volume is decreased.

2. Liquid pump appliance according to claim 1,
wherein the pump tube (7) and the first and the second non-return valve (5, 6) are arranged between the bottom of the supply vessel (1) and the discharge tube (4).

3. Liquid pump appliance according to claim 2,
wherein the pump tube (7) extends with its length substantially perpendicularly to the length extension of the liquid pump appliance.

4. Liquid pump appliance according to claim 1,
comprising an actuation button (12) which is connected with the piston (9), for manually sliding the piston (9) within the pump tube (7) in a first direction.

5. Liquid pump appliance according to claim 4,
comprising a pressure spring or a tension spring (8) which is acting on the piston (9) or the actuation button (12), and by means of which the piston (9) is slidable within the pump tube (7) in a second direction which is opposite to the first direction.

6. Liquid pump appliance according to claim 4,
wherein the actuation button (12) is connected with the piston (9) by means of a screw thread mechanism which comprises a threaded rod (10) and a threaded sleeve (11) which are twistable against each other, for varying the length of the screw thread mechanism between the actuation button (12) and a first stop (91a) for the actuation button (12), and, by this, for adjusting a maximum sliding path of the piston (9).

7. Liquid pump appliance according to claim 1,
wherein the volume for the fluid within the pump tube (7) is located between the piston (9) and a first end or an opposite second end (91a; 92) of the pump tube (7).

8. Liquid pump appliance according to claim 1,
wherein the first non-return valve (5) is fluid-connected with an opening (31) in the bottom surface of the supply vessel (1).

9. Liquid pump appliance according to claim 1,
wherein the supply vessel (1) comprises a supply vessel piston (2) which is hermetically guided within the vessel such that it rests on the fluid and sinks with falling fluid level.

10. Liquid pump appliance according to claim 9,
wherein the surface of the supply vessel piston (2) which is opposite to the bottom surface of the supply vessel (1) is adapted with respect to its shape and course to the bottom surface of the supply vessel (1).

11. Liquid pump appliance according to claim 1,
wherein the supply vessel (1) comprises an opening (3) for the supply of air when fluid is removed from the supply vessel (1) through the dispense device.

## Revendications

1. Accessoire de pompage de liquide pour distribution ou injection dosée de fluides, comprenant un réservoir d'alimentation (1) pour le fluide (M) et un dispositif de distribution du fluide (M), dans lequel le dispositif de distribution comprend un tube de pompage (7),
**caractérisé en ce qu'**un volume destiné au fluide (M) est défini dans le tube de pompage (7) par l'intermédiaire d'un piston (9) qui est guidé en étant hermétiquement étanche dans le tube de pompage, le piston (9) pouvant coulisser le long du tube de pompage (7) pour modifier ce volume, et le fluide (M) étant extrait du réservoir d'alimentation (1) à travers un premier clapet anti retour (5) à l'intérieur de ce volume lorsque que le volume est augmenté, et est distribué à partir de ce volume à travers un second clapet anti retour (6) et à travers un tube d'évacuation (4) à l'extérieur de l'accessoire de pompage de liquide lorsque le volume est diminué.

2. Accessoire de pompage de liquide selon la revendication 1,
dans lequel le tube de pompage (7) et le premier et le second clapet anti retour (5, 6) sont agencés entre le fond du réservoir d'alimentation (1) et le tube d'évacuation (4).

3. Accessoire de pompage de liquide selon la revendication 2,
dans lequel le tube de pompage (7) s'étend en ayant sa longueur sensiblement perpendiculaire à l'étendue de la longueur de l'accessoire de pompage de liquide.

4. Accessoire de pompage de liquide selon la revendication 1,
comprenant un bouton d'actionnement (12) qui est relié au piston (9), pour faire coulisser manuellement le piston (9) dans le tube de pompage (7) dans une première direction.

5. Accessoire de pompage de liquide selon la revendication 4,
comprenant un ressort de pression ou un ressort de traction (8) qui agit sur le piston (9) ou le bouton d'actionnement (12), et par l'intermédiaire duquel le piston (9) peut coulisser dans le tube de pompage (7) dans une seconde direction qui est opposée à la première direction.

6. Accessoire de pompage de liquide selon la revendication 4,
dans lequel le bouton d'actionnement (12) est relié au piston (9) par l'intermédiaire d'un mécanisme à vis et filetage qui comprend une tige filetée (10) et un manchon fileté (11) qui peuvent tourner à l'encontre l'un de l'autre, pour modifier la longueur du mécanisme à vis et filetage entre le bouton d'actionnement (12) et une première butée (91a) pour le bouton d'actionnement (12), et, par ceci, pour ajuster un trajet de coulissement maximum du piston (9).

7. Accessoire de pompage de liquide selon la revendication 1,
dans lequel le volume destiné au fluide dans le tube de pompage (7) est situé entre le piston (9) et une première extrémité ou une seconde extrémité opposée (91a ; 92) du tube de pompage (7).

8. Accessoire de pompage de liquide selon la revendication 1,
dans lequel le premier clapet anti retour (5) est relié hydrauliquement à une ouverture (31) située dans la surface de fond du réservoir d'alimentation (1).

9. Accessoire de pompage de liquide selon la revendication 1,
dans lequel le réservoir d'alimentation (1) comprend un piston de réservoir d'alimentation (2) qui est guidé de manière hermétique dans le réservoir de telle sorte qu'il soit en appui sur le fluide et s'enfonce avec un niveau de fluide diminuant.

10. Accessoire de pompage de liquide selon la revendication 9,
dans lequel la surface du piston(2) du réservoir d'alimentation qui est opposée à la surface de fond du réservoir d'alimentation (1) est adaptée en ce qui concerne sa forme et sa course à la surface de fond du réservoir d'alimentation (1).

11. Accessoire de pompage de liquide selon la revendication 1,
dans lequel le réservoir d'alimentation (1) comprend une ouverture (3) destinée à l'alimentation d'air lorsque du fluide est enlevé du réservoir d'alimentation (1) à travers le dispositif de distribution.
